# EUROPÄISCHE PATENTSCHRIFT

(11) **EP 0 650 705 B1**
(45) Veröffentlichungstag und Bekanntmachung des Hinweises auf die Patenterteilung: **10.04.1996**
(21) Anmeldenummer: 94116728.0
(22) Anmeldetag: 24.10.1994
(51) Int. Cl.: A61F 2/24

(54) **Doppelflügel-Herzklappenprothese**
Two leaflet prosthetic heart valve
Prothèse valvulaire cardiaque à deux clapets

(30) Priorität: 28.10.1993 DE 4336899
(43) Veröffentlichungstag der Anmeldung: 03.05.1995
(73) Patentinhaber: INOCOR GmbH, D-82319 Starnberg (DE)
(72) Erfinder: Frey, Rainer H., D-82319 Starnberg (DE)
(74) Vertreter: Brehm, Hans-Peter, Dr., Dipl.-Chem.

(56) Entgegenhaltungen:
- EP-A- 0 079 844
- WO-A-89/02254

## Beschreibung

Die Erfindung betrifft eine Doppelflügel-Herzklappenprothese nach dem Oberbegriff des Anspruches 1.

Bei einer solchen zweiflügeligen, mechanischen Herzklappenprothese werden die Klappenflügel allein durch den Strömungsdruck des anströmenden Blutes zwischen einer Offen-Stellung und einer Schließ-Stellung verschwenkt. Diese Kunstklappe ist insbesondere zur dauerhaften Implantation am Menschen bestimmt und kann hier insbesondere in der Aortenposition und in der Mitralposition eingesetzt werden.

Im einzelnen betrifft die Erfindung eine Doppelflügel-Herzklappenprothese mit einem im wesentlichen kreisrunden Klappenring, dessen Innenumfang einen Strömungskanal für die Blutströmung begrenzt. Diese Herzklappenprothese weist zwei im wesentlichen halbmondförmige Klappenflügel auf, die beide und unabhängig voneinander schwenkbar an einer gemeinsamen Achse angelenkt sind, die am Klappenring gehalten ist. Diese Klappenflügel können - abhängig von der Strömungsrichtung des Blutes - eine Offen-Stellung im wesentlichen vertikal zur Klappenringebene oder eine Schließ-Stellung im wesentlichen in Richtung der Klappenringebene einnehmen, in welcher beide Klappenflügel mit ihren zusammengenommenen Flächen den Strömungskanal nahezu vollständig verschließen.

Eine Doppelflügel-Herzklappenprothese dieser Art ist aus der deutschen Offenlegungsschrift 31 28 704 oder aus der Internationalen Patentanmeldung WO 89/02254 bekannt. Die bekannten Doppelflügel-Prothesen sehen insoweit einen symmetrischen Aufbau vor, als die beiden Klappenflügel einer Prothese im Umriß eine gleiche Fläche bzw. Flächengröße aufweisen und symmetrisch bezüglich einer Normalmittelebene zur Klappenringebene angeordnet sind, das heißt, eine Längsmittelachse der einzigen Achse, an der die beiden Klappenflügel angelenkt sind, fluchtet mit einem Durchmesser des Klappenringes. Die Offenstellung der Klappenflügel wird durch Anschläge begrenzt.

Die Entwicklung der mechanischen Herzklappenprothesen ging von den Kugel-Käfig-Prothesen ("Starr-Edwards Herzklappe") aus und führte über die Kippscheiben-Prothesen ("Björk-Shiley Herzklappe", "Medtronic-Hall Herzklappe", "Omnicarbon Herzklappe") zu den heute überwiegend eingesetzten Doppelflügel-Prothesen ("St. Jude Medical Herzklappe", "Edward-Duromedics Herzklappe", Carbomedics Herzklappe" und "Sorin-Bicarbon Herzklappe"). Jeder Klappenflügel weist eine eigene Befestigungs- und/oder Führungseinrichtung auf. In der Offen-Stellung sind die beiden Klappenflügel im wesentlichen parallel und im Abstand zueinander angeordnet. Dies führt dazu, daß im wesentlichen drei Strömungszonen auftreten; eine Strömungszone zwischen den Klappenflügeln und je eine Strömungszone zwischen Klappenring und Anströmfläche des Klappenflügels. Mit dieser Anordnung konnte eine Verminderung des Druckgradienten, eine bessere Ansprache der Klappenflügel auf eine Änderung von Blutdruck und Blutströmungsrichtung mit einer entsprechenden Verminderung der Regurgitation und eine Annäherung der Hämodynamik an die Physiologie der natürlichen Klappenapparate erzielt werden. Problematisch ist jedoch weiterhin eine Thrombembolie-Gefahr, die eine fortdauernde kontrollierte Verabreichung von Anticoagulentia erfordert und eine hartnäckige Hämolyse, die häufig Jahre nach dem Klappenersatz zunehmen kann; vgl. in diesem Zusammenhang auch D. Horstkotte, C. Aul und L. Seipel "Einfluß von Klappentyp und Klappenfunktion auf die chronische intravasale Hämolyse nach alloprothetischem Mitral- und Aortenklappenersatz" in Z. Kardiol., 72, (1983), 119.

Eine Hämolyse beruht auf einer Schädigung der verformbaren Blutbestandteile (Erythrozyten) und kann durch LDH-Anstieg bzw. durch Haptoglobinabfall im Plasma nachgewiesen werden. Zu wesentlichen Ursachen für eine Hämolyse gehören hohe Strömungsgeschwindigkeiten (Jetströmung), Turbulenzen und Wirbelströmungen, die hohe Schubspannungen verursachen. Die letzteren treten insbesondere in den Grenzschichten zwischen benachbarten Strömungszonen mit unterschiedlichem Geschwindigkeitsprofil auf. Ferner verursacht ein asynchrones Öffnungs- und Schließverhalten der beiden Klappenflügel einer Kunstklappe Turbulenzen und Wirbelströmungen. Ferner kann eine Annäherung des Klappenflügels an den Klappenring während des Schließvorganges eine mechanische Schädigung der Erythrozyten verursachen. Speziell in der Aortenposition können randnahe Strömungszonen mit hoher Strömungsgeschwindigkeit eine Schädigung des Endothels am Aortenbogen verursachen. Ferner muß bei einer symmetrischen Doppelflügel-Aortenprothese wegen langer Verschlußzeit und asynchroner Schließbewegung der beiden Klappenflügel mit hoher Regurgitation gerechnet werden. In der Mitralposition kann mit einer symmetrisch aufgebauten Doppelflügelprothese kein physiologisches Strömungsprofil im Ventrikel erzielt werden, weil sich die beiden Randströmungen gegenseitig behindern. Das Auswaschen des Ventrikels ist ungenügend. Auch durch eine Drehung der symmetrischen Kunstklappe in der Klappenringebene kann der Chirurg diesen Mangel nicht beheben.

Es sind Verfahren entwickelt worden, die es mit Hilfe von gelösten, kurzzeitig erregten photochromen Farbstoffmolekülen ermöglichen, die Strömungsverhältnisse in einer pulsierenden Flüssigkeit stromabwärts zu einer Kunstklappe sichtbar zu machen; vgl. etwa Yurechko, V.N. et al. in ASAIO Transactions, 1989, 35, 218-221, oder in The International Journal of Artificial Organs, No. 6, 1993, 29-33. Diese Untersuchungen bestätigen beispielsweise distal (stromabwärts) zu einer geöffneten "St. Jude Medical Herzklappe" ein Strömungsprofil mit drei Strömungszonen mit unterschiedlichen Geschwindingkeitsprofilen.

Mit dem genannten Verfahren von Yurechko, V. N. konnte im Rahmen der vorliegenden Erfindung gezeigt werden, daß stromabwärts zu einer Doppelflügelprothese, deren beide Klappenflügel an einer gemeinsamen Achse angelenkt sind, vergleichsweise einfache Strömungsverhältnisse auftreten. In der Offen-Stellung tritt stromabwärts zu den beiden V-förmig angeordneten Klappenflügeln eine Stagnationszone auf, an die sich auf beiden Seiten je eine Strömungszone anschließt. Die Ausdehnung der Stagnationszone kann durch den Öffnungswinkel der Klappenflügel und die Konfiguration der Klappenflügel-Anströmseite beeinflußt werden. Durch Abstimmung dieser Parameter kann eine relativ kleine Stagnationszone erzielt werden. Damit läßt dieser Prothesentyp ein einfacheres Strömungsprofil erwarten als andere Doppelflügelprothesen. Ein Strömungsprofil mit lediglich zwei Strömungszonen erzeugt weniger Turbulenzen und damit geringere Schubspannungen auf die Erythrozyten. Der notwendige Öffnungswinkel zwischen den beiden V-förmig angeordneten Klappenflügeln beschleunigt das Schließverhalten. Ein schnelleres Schließverhalten verringert den Energieverlust.

Ferner ermöglicht diese Bauart eine einfache, stabile und zuverlässige Befestigung der beiden Klappenflügel an einer gemeinsamen Achse. Die Achse kann mit ausreichendem Durchmesser ausgeführt werden und sicher am Klappenring gelagert werden. Die Achse ist durch Hülsenabschnitte geführt, die einstückig am Klappenflügel angeformt sind. Dadurch wird hohe Stabilität und Zuverlässigkeit erzielt. Ein Herausfallen eines Klappenflügels ist unmöglich; im Gegensatz zu bekannten Vorfällen, beispielsweise bei "St. Jude Medical Herzklappen" (vgl. Journal of Thorac. Cardiovasc. Surgery, Nr. 31 (1983) und Nr. 86 (1983), oder bei "Edward-Duromedics Herzklappen" (vgl. Journal of Thorac. Cardiovasc. Surgery, Nr. 97 (1984), S. 90-94).

Die anatomischen Verhältnisse erlauben beim Einbau einer Kunstklappe nur wenig Spielraum. Zumeist wird der Nahtring am natürlichen Klappenring oder an Resten der exerzierten Klappen befestigt. Die Lage der Koronarostien und des aortalen Segels verursachen weitere Beschränkungen beim Einsetzen einer Aortenprothese. In der Mitralposition muß der Jetstrom beim Schließen der Aortenklappe auf den Schließvorgang einer Mitralprothese beachtet werden.

Wegen der exzentrischen Aufhängung der Kippscheibe liefert eine Kippscheibenprothese notwendigerweise ein asymmetrisches, distales Strömungsprofil, das der Chirurg bei der Orientierung der Klappe ausnutzen konnte zur Annäherung an die physiologischen Verhältnisse; vgl. beispielsweise Viking O. Björk und Dan Lindblom, "The Monostrut Björk-Shiley Heart Valve", in Journal of the American College of Cardiology, Vol. 6, No. 5, 1142-1148 (Nov. 1985). In diesem Beitrag wird sowohl in der Aortenposition wie in der Mitralposition eine bevorzugte Ausrichtung der Kippscheibe angegeben mit entsprechenden Auswirkungen auf die Strömungsvolumina in den Abschnitten der Strömungskanalquerschnitte in der Klappenringebene und auf das distale Strömungsprofil. Bei symmetrisch aufgebauten Doppelflügel-Klappenprothesen besteht diese Möglichkeit nicht.

Wie oben dargelegt, weist eine Doppelflügelprothese, insbesondere eine Doppelflügelprothese, bei welcher die beiden Klappenflügel an einer gemeinsamen Achse angelenkt sind, grundsätzlich gewisse Vorteile gegenüber einer Kippscheibenprothese auf. Es wäre jedoch wünschenswert, zusätzlich zur Auswahl der Größe (Klappenring-Durchmesser) auch bei einer solchen Doppelflügel-Kunstklappe eine weitere Möglichkeit zu schaffen, mit welcher der Chirurg die Strömungsverhältnisse beeinflussen kann.

Davon ausgehend, besteht die Aufgabe der vorliegenden Erfindung darin, eine Doppelflügel-Herzklappenprothese der angegebenen Art (bei welcher die beiden Klappenflügel an einer gemeinsamen Achse angelenkt sind) bereitzustellen, die dem Chirurgen eine zusätzliche Möglichkeit zur Beeinflussung der Strömungsverhältnisse bietet.

Ausgehend von einer Doppelflügel-Herzklappenprothese mit einem im wesentlichen kreisrunden Klappenring, dessen Innenumfang einen Strömungskanal für die Blutströmung begrenzt, und
mit zwei im wesentlichen halbmondförmigen Klappenflügeln, die beide und unabhängig voneinander schwenkbar an einer gemeinsamen Achse angelenkt sind, die am Klappenring gehalten ist, wobei beide Klappenflügel - abhängig von der Blutströmungsrichtung - eine Offen-Stellung im wesentlichen vertikal zur Klappenringebene oder eine Schließ-Stellung im wesentlichen in Richtung der Klappenringebene einnehmen können, in welcher beide Klappenflügel mit ihren zusammengenommenen Flächen den Strömungskanal nahezu vollständig verschließen,
ist die erfindungsgemäße Lösung obiger Aufgabe dadurch gekennzeichnet, daß
die Achse exzentrisch, das heißt parallel und im Abstand zu einem Kreisdurchmesser am Klappenring angeordnet ist; und
der eine Klappenflügel eine größere Fläche aufweist als der andere Klappenflügel.

Vorzugsweise ist vorgesehen, daß der flächenmäßig größere Klappenflügel etwa 55 % bis 80 % des Strömungskanalquerschnittes verschließt und der flächenmäßig kleinere Klappenflügel etwa 45 % bis 20 % des Strömungskanalquerschnittes verschließt. Sofern der flächenmäßig größere Klappenflügel weniger als 55 % des Strömungskanalquerschnittes verschließt, ist der mit der Erfindung erzielte Effekt zu gering. Sofern der flächenmäßig kleinere Klappenflügel weniger als 20 % des Strömungskanalquerschnittes verschließt, treten in diesem Abschnitt distal zur Kunstklappe Rezirkulationsareale und Bereiche mit verminderter oder aufgehobener Strömung auf, welche die Entstehung von fibrotischem und thrombotischem Material begünstigen. Es besteht die Gefahr einer höheren Thrombembolierate.

Noch weiter bevorzugt ist vorgesehen, daß der flächenmäßig größere Klappenflügel etwa 60 bis 68 % des Strömungskanalquerschnittes verschließt und der flächenmäßig kleinere Klappenflügel etwa 40 bis 32 % des Strömungskanalquerschnittes verschließt. Hier wird einerseits ein für die Anpassung an die jeweiligen anatomischen und physiologischen Verhältnisse günstiges asymmetrisches distales Strömungsprofil erzielt. Andererseits wird ein sicheres, schnelles Schließverhalten mit geringerem Regurgitationsvolumen erzielt.

Eine erfindungsgemäße, asymmetrisch ausgebildete Doppelflügelprothese kann in jeder Klappenposition eingesetzt werden. Vorzugsweise wird eine erfindungsgemäße Kunstklappe in der Aortenposition oder in der Mitralposition eingesetzt.

Beim Einsatz als Aortenprothese ist der flächenmäßig größere Klappenflügel dazu bestimmt, den dorsalen Abschnitt des Strömungskanalquerschnittes zu verschließen, und der flächenmäßig kleinere Klappenflügel ist dazu bestimmt, den ventralen Abschnitt des Strömungskanalquerschnittes zu verschließen. Von Björk und Lindblom wird für eine Kippscheibenprothese in der Aortenposition die entgegengesetzte Anordnung angegeben. Dies mag auch darauf beruhen, daß die Kippscheibe in der Offen-Stellung wesentlich weiter über die Klappenringebene hinaussteht. Demgegenüber weist die erfindungsgemäße Prothese in der Offen-Stellung eine geringere Bauhöhe auf und erfordert somit weniger freien Raum. Bei der erfindungsgemäß vorgesehenen Anordnung in der Aortenposition kann das zu fördernde Blutvolumen besser an die Anatomie der Aorta angepaßt werden; ferner werden Endothelschädigungen vermieden. Beim Einsatz als Mitralprothese ist der flächenmäßig größere Klappenflügel dazu bestimmt, den dorsalen Abschnitt des Strömungskanalquerschnittes zu verschließen, und der flächenmäßig kleinere Klappenflügel ist dazu bestimmt, den ventralen Abschnitt des Strömungskanalquerschnittes zu verschließen. Diese Anordnung begünstigt eine Auswaschströmung im linken Ventrikel.

Die asymmetrische Ausbildung und Anordnung der Klappenflügel führt dazu, daß in der Offen-Stellung ein größerer Abschnitt und ein kleinerer Abschnitt des Strömungskanalquerschnittes gebildet werden, durch den Blut strömt. Angrenzend an den flächenmäßig größeren Klappenflügel tritt eine volumenmäßig größere Blutströmung auf, und angrenzend an den flächenmäßig kleineren Klappenflügel tritt eine volumenmäßig kleinere Blutströmung auf. Ferner wird sich bei den hier vorgegebenen Abmessungen stromabwärts zum kleineren Abschnitt ein Strömungsprofil mit einer kleineren mittleren Strömungsgeschwindigkeit aufbauen als stromabwärts zum größeren Abschnitt. Der Chirurg kann diesen Umstand gezielt dazu nutzen, das asymmetrische Strömungsprofil der Physiologie anzupassen. Hierdurch kann die Gefahr einer Bildung von Zonen mit Wirbelströmung oder die Größe von Totwasserzonen vermindert werden. Weiterhin wird das Endothel an den stark gekrümmten Innenwandabschnitten geschont, weil dort geringere Blutströmungsgeschwindigkeiten auftreten. Wegen der geringeren Schubspannung wird die Hämolyse vermindert.

Ersichtlich wird dem Chirurgen ein weiterer Freiheitsgrad an die Hand gegeben, durch einfache Drehung des Klappenringes in der Klappenringebene die distal zur Kunstklappe auftretenden Strömungsverhältnisse zu beeinflussen. Insbesondere können diese Strömungsverhältnisse besser an die Anatomie und Physiologie des jeweiligen Einzelfalles angepaßt und damit die Gefahr einer Hämolyse vermindert werden.

Aus dem Dokument EP 0 079 844 B1 ist zwar eine Kunstklappe mit zwei Klappenflügeln bekannt, die ungleiche Oberflächen haben. Jedoch weist jeder Klappenflügel eine eigene Achse auf, um welche der jeweilige Klappenflügel schwenkbar am Klappenring angelenkt ist, und diese beiden Achsen sind nicht in einer gemeinsamen Ebene angeordnet, was notwendigerweise zwei getrennte Achsen erfordert. Es soll das Klappenöffnungsverhalten verbessert und damit sehr schnell eine perfekt homogene Zone der Blutströmung erzielt werden. Ein asymmetrisches Strömungsprofil wird gerade nicht angestrebt.

Vorteilhafte Ausgestaltungen und Weiterbildungen der Erfindung ergeben sich aus den Unteransprüchen.

Entsprechend der vorliegenden Erfindung erfolgt die asymmetrische Anordnung der Klappenflügel in der Weise, daß die Achse exzentrisch, d.h. parallel und im Abstand zu einem Durchmesser eines Kreises angeordnet ist, der durch den Klappenring definiert ist. In diesem Falle bildet die Achse, genauer die Längsmittelachse der mechanisch wirksamen Achse (Welle) eine Sehne an diesem Kreis. Der Abstand der Sehne zu einem parallelen Durchmesser an diesem Kreis wird als Exzentrizität bezeichnt. Vorzugsweise kann die Exzentrizität, d.h. der Abstand einer Längsmittelachse der Achse zu einem parallelen Durchmesser etwa 4 % bis 22 % des Innendurchmessers des Klappenringes betragen. Mit einer solchen Exzentrizität wird das gewünschte Verhältnis von flächenmäßig größerem zu flächenmäßig kleinerem Klappenflügel ermöglicht.

Jeder Endabschnitt der Achse ist in je einem Achslager gelagert, das im Klappenring ausgebildet ist. Typischerweise sind diese Achslager als Aussparungen oder durchgehende Bohrungen im Klappenring ausgebildet.

Nach einer weiteren vorteilhaften Ausgestaltung kann vorgesehen sein, diese Achslager als längliche Aussparungen auszubilden, in denen die Achsen-Endabschnitte schwimmend, d.h. verschieblich oder verstellbar gelagert sind. Der Weg für eine solche Verstellung der Achsen-Endabschnitte innerhalb der länglichen Achslager kann etwa 2 bis 4 mm betragen. Vorzugsweise erstreckt sich die Richtung der länglichen Achslager-Aussparung von links unterhalb nach rechts oberhalb der Klappenringebene; im Falle einer Aortenprothese also von kaudal nach kranial und von ventral nach dorsal. Mit einer solchen schwimmenden bzw. verschieblichen Achslagerung werden Druckgradienten innerhalb der Blutströmung abgebaut und das Öffnungs- und Schließverhalten der Klappenflügel verbessert. Es sind solche Abmessungen von Achsen-Endabschnitt und Achslager-Aussparung vorgesehen, daß eine stetige Spülung mit frischem Blut am Achslager gewährleistet ist. Der Klappenring kann im Bereich der beiden Achslager Abschnitte mit größerer Höhe (sogenannte Ohren) aufweisen, um genügend Platz für die länglichen und schräg ausgerichteten Achslager-Aussparungen zu schaffen.

Nach einer weiteren vorteilhaften Ausgestaltung kann ein derartiges, längliches, gegenüber der Blutströmungsrichtung geschräg gestelltes Achslager vorzugsweise an einem Klappenring vorgesehen werden, der am Innenumfang, zumindest im Bereich der Achslager,eine Konfiguration oder Kontur aufweist, die sich im Strömungsrichtung erweitert. Hierdurch kann erreicht werden, daß bei einer Wanderung der Achse in Strömungsrichtung auch die Weite (Spaltbreite, Abstand) zwischen dem Innenumfangsabschnitt des Klappenringes und dem benachbarten Randabschnitt des Klappenflügels zunimmt. Durch den so größer werdenden Spalt zwischen Klappenflügelrand und Klappenring-Innenumfang kann mehr Blut strömen. Die verbesserte Blutströmung vermindert die Gefahr einer Thrombenbildung, die gerade bei bekannten Doppelflügelprothesen im Bereich der Befestigungsstellen notorisch groß ist.

Nach einer weiteren vorteilhaften Ausgestaltung kann die Achse zweiteilig ausgebildet sein und aus zwei Achsenabschnitten bestehen. Jeder Achsenabschnitt weist an einem Ende einen verdickten Kopf auf, der im Achslager gehalten und gegen Herausrutschen gesichert ist. Die beiden anderen Endabschnitte der Achsenabschnitte sind als ineinandersteckbare(r) Stift und Hülse ausgebildet. Zusätzlich kann im Bereich des Ineinandergreifens von Stift und Hülse eine Rastverbindung in Form einer umlaufenden Nut- und Feder-Anordnung ausgebildet sein. Die zweiteilige Ausbildung der Achse erleichtert die Herstellung und den Zusammenbau der Prothese, weil Biegebeanspruchungen vermieden werden, die zu mechanischen Spannungen und Haarrissen führen könnten, welche die verwertbare Lebensdauer der Prothese beeinträchtigen.

Jeder Klappenflügel hat eine im wesentlichen halbmondförmige Gestalt und wird von einem bogenförmigen, insbesondere halbkreisförmigen Randabschnitt sowie von einem geraden Randabschnitt begrenzt. Im Bereich des geraden Randabschnittes sind einstückig ein oder zwei Hülsenabschnitte angeformt, durch deren Bohrung die Achse geführt ist. Bei einer ersten Ausführungsform erstreckt sich dieser Hülsenabschnitt vom Außenende des geraden Randabschnittes bis etwa zur Klappenflügelmitte. Beide Klappenflügel werden parallel und einander gegenüber derart angeordnet, daß eine fluchtende Bohrung der beiden Hülsenabschnitte resultiert. Die Achse ist durch diese fluchtende Bohrung geführt. Bei einer anderen Ausführungsform kann - vorzugsweise an dem flächenmäßig größeren Klappenflügel - in den beiden Endbereichen des geraden Randabschnittes je ein Hülsenabschnitt angeformt sein, zwischen denen eine Lücke besteht. An dem anderen Klappenflügel - vorzugsweise dem flächenmäßig kleineren Klappenflügel - ist im Mittelbereich des geraden Randabschnittes ein Hülsenabschnitt angeformt, der in diese Lücke paßt. Nachdem die beiden Klappenflügel einander gegenüber angeordnet sind, bilden sämtliche Hülsenabschnitte eine fluchtende Bohrung, durch welche die Achse geführt ist. Mit Hilfe dieser Hülsenabschnitte wird eine scharnierartige Anlenkung der beiden Klappenflügel an einer einzigen Achse erzielt. Diese scharnierartige Anlenkung liefert eine sichere und zuverlässige Befestigung der beiden Klappenflügel am Klappenring.

Vorzugsweise sind der Außendurchmesser der Achse und der Innendurchmesser der Bohrung der Hülsenabschnitte derart bemessen und aufeinander abgestimmt, daß einerseits eine leichtgängige Verschwenkbarkeit der Klappenflügel um die Achse gewährleistet ist, und daß andererseits Blut im wesentlichen nicht in die Bohrung der Hülsenabschnitte eindringen kann. Hierzu werden die Oberflächen auf einige Mikrometer genau bearbeitet. Der Zutritt von Blut zu den bei einer Klappenflügel-Verschwenkung reibenden Oberflächen zwischen Achse und Hülsenabschnitt-Innenumfang ist unterbunden und damit eine Schädigung von Blutbestandteilen einschließlich einer Klott-Bildung auf ein Minimum herabgesetzt. Auch fehlen Totwassergebiete, die bei anderen, bekannten Klappen häufig Ausgangspunkt für eine Klott-Bildung sind.

Die Stirnflächen der Hülsenabschnitte sind senkrecht zu der Achse ausgerichtet. Dies gewährleistet eine gegenseitig unabhängige Verschwenkung der beiden Klappenflügel.

Die Klappenflügel sind starr und weisen typischerweise im Bereich der flügelartigen Schließkörper eine Wandstärke zwischen etwa 0,5 und 2 mm auf. In Richtung zum Hülsenabschnitt hin kann die Wandstärke zunehmen, so daß insbesondere die Anströmfläche der Klappe tangential und stetig in die Krümmung des Hülsenabschnittes übergeht. Darüberhinaus kann vorzugsweise eine konvexe Kontur bzw. Konfiguration jeder Anströmfläche der Klappenflügel vorgesehen sein. Es wird eine glatte, dem Blutstrom wenig Widerstand entgegensetzende Konfiguration erhalten. Eine konvexe Kontur der Anströmflächen vermindert die Ausdehnung der Stagnationszone distal zu den geöffneten, V-förmig angeordneten Klappenflügeln. Die wesentlichen vom Blutstrom kontaktierten Flächen sind leicht gekrümmt bzw. gewölbt und aufgrund ihrer Anordnung und Konfiguration optimal an die Blutströmung angepaßt. Sämtliche Flächen sind ständig umspült; Totwassergebiete treten nicht auf. Es fehlen Kanten und Aussparungen, die zu Verwirbelungen des Blutstromes bzw. zum Absetzen von Fibrinogen führen könnten. Daher ist eine geringe Thrombembolirate zu erwarten.

In der Schließ-Stellung befinden sich die Klappenflügel im wesentlichen in der Ebene des Klappenringes. Am Innenumfang des Klappenringes können ein oder mehrere Anschläge ausgebildet sein, welche ein Hindurchschwingen der Klappenflügel durch die Klappenebene, beispielsweise in der Aortenposition, nach kaudal verhindern. Beispielsweise können diese Anschläge als am Innenumfang des Klappenrings umlaufende Stufe ausgebildet sein, auf welcher der halbkreisförmige Rand der Klappenflügel aufliegen kann. Vorzugsweise sind lediglich Stufenabschnitte in je einer begrenzten Innenumfangszone des Klappenringes vorgesehen, die sich auf beiden Seiten zu einem Achslager erstrecken. Vorzugsweise weist jeder Stufenabschnitt nur eine Länge von etwa 3 bis 6 mm auf und erstreckt sich zu beiden Seiten eines Achslagers am Innenumfang des Klappenringes. Diese Stufenabschnitte sollen homogen und stetig aus der Innenumfangswand des Klappenringes heraustreten und an ihrem Ende wieder homogen und stetig in diese Innenumfangswand übergehen. In diesem Bereich tritt nur eine geringe Relativgeschwindigkeit der bewegten Klappenflügel-Randabschnitte bezüglich der feststehenden Stufenabschnitte auf. Es wird eine Cavitation vermieden, die zur Erosion der Klappenflügelkante führen könnte. Ferner ist die Aufprallfläche der Klappenflügel-Randabschnitte auf den Stufenabschnitten minimal gehalten.

In der Offen-Stellung nehmen die Klappenflügel eine Anordnung im wesentlichen parallel zu und in der Richtung des Blutstromes ein. Die Klappenflügel nehmen im wesentlichen eine V-Stellung ein, die durch einen Klappenflügel-Öffnungswinkel definiert ist. Im Rahmen dieser Unterlagen ist der Klappenflügel-Öffnungswinkel begrenzt durch zwei Geraden, die ausgehend von der Längsmittelachse der Achse durch die beiden Scheitel der halbkreisförmigen Klappenflügel führen. Im Falle einer in vitro Nachbildung der Aortenposition konnten mit dem eingangs genannten Verfahren von V. N. Yurechko zur Sichtbarmachung der Strömungsverhältnisse in dieser Offen-Stellung im wesentlichen drei verschiedene Strömungszonen verifiziert werden. In den Aortenbulben (Sinus) tritt je eine Wirbelströmung auf. Beidseitig zu den beiden Klappenflügeln tritt je eine Strömungszone auf. Distal zu den V-förmig angeordneten Klappenflügeln tritt eine Stagnationszone auf. Die Größe dieser Stagnationszone ist im wesentlichen proportional zum Klappenflügel-Öffnungswinkel. Einerseits soll die Stagnationszone möglichst klein gehalten werden. Andererseits treten bei einem zu kleinen Klappenflügel-Öffnungswinkel Unsicherheiten und Verzögerungen im Ansprechen der Klappenflügel auf eine Umkehr der Blutströmungsrichtung auf, die letztlich wieder zur Einnahme der Schließ-Stellung führt.

Nach einer vorteilhaften Ausgestaltung sollen die an einer gemeinsamen Achse angelenkten Klappenflügel in der Offen-Stellung einen Klappenflügel-Öffnungswinkel von etwa 10° bis 40° einnehmen. Bei einem kleineren Öffnungswinkel ist das Ansprechverhalten der Klappenflügel auf eine Umkehr der Blutströmungsrichtung ungenügend. Bei einem größeren Öffnungswinkel nimmt die Stagnationszone eine zu große Ausdehnung ein. Noch weiter bevorzugt ist ein Klappenflügel-Öffnungswinkel von etwa 12 bis 18°, weil hier eine besonders kleine Stagnationszone bei sicherem Schließverhalten erhalten wird.

Vorzugsweise sind am Innenumfang des Klappenringes auch Anschläge angeformt, welche ein Hinausschwingen der Klappenflügel über die vorgesehene Position in deren Offen-Stellung verhindern. Diese Anschläge können beispielsweise vom oberen Abschnitt des Klappenringes nach innen vorstehende Stifte sein, die sich zwischen den beiden Klappenflügeln in deren Offen-Stellung befinden. Hierbei ist zu beachten, daß die Klappenflügel in der Offenstellung nicht notwendigerweise eine symmetrische Position benachbart zu einer Senkrechten auf die Klappenringebene einnehmen müssen. Vielmehr sollen die Klappenflügel in der Offen-Stellung eine Position einnehmen, welche im wesentlichen den Strömungslinien der Blutströmung entspricht.

Häufig ist es zweckmäßig, an dem zum flächenmäßig kleineren Klappenflügel benachbarten Abschnitt des Klappenringes eine größere Höhe vorzusehen als am restlichen Klappenring. Zusätzlich kann dieser, eine größere Höhe aufweisende Klappenringabschnitt mit einer leicht nach innen gerichteten Krümmung versehen werden. Der Einlauf der Blutströmung in den anschließenden, gekrümmten Wandabschnitt der Kammer oder des Gefäßes wird verbessert.

Sowohl der Klappenring wie die beiden Klappenflügel bestehen aus dauerhaften, bio-verträglichen Materialien, die mit einer die Blutgerinnung hemmenden Oberfläche ausgerüstet sind. Das bevorzugte Material für die Oberflächen ist pyrolytischer Kohlenstoff (Polycarbon). Typischerweise enthalten der Klappenring und/oder die Klappenflügel versteifende oder verstärkende Einlagen, die mit pyrolytischem Kohlenstoff beschichtet sind. Diese Einlagen können beispielsweise aus Edelstahl, aus Titan oder aus einer Al/Ti-Legierung oder aus einer Co-Legierung (wie etwa HAYNES 25) oder aus anderen bekannten geeigneten Materialien bestehen. Ein besonders bevorzugtes Material wird durch Sputtern eines Titan-Targets mit Kohlenstoff erhalten. Es wird eine mehrschichtige Struktur erhalten, wobei die Deckschicht aus pyrrolytischem Kohlenstoff über Zwischenschichten aus Titancarbid(en) an einen Kern aus metallischem Titan gebunden ist. Die Haftung und Dauerhaftigkeit der Deckschicht aus pyrrolytischem Kohlenstoff ist besonders gut. Der Klappenring weist typischerweise eine Höhe zwischen etwa 3 und 6 mm und einen Strömungskanaldurchmesser - je nach Anwendungsfall und Alter des Patienten - zwischen etwa 12 und 29 mm auf. Die Klappenflügel weisen angepaßte Abmessungen auf, um mit ihren zusammengenommenen Flächen den vom Klappenring begrenzten Strömungskanal nahezu zu verschließen. Auch in der Schließ-Stellung soll eine geringfügige Blutströmung gewährleistet sein. Der Innenumfang des Klappenrings ist vorzugsweise konvex ausgebildet. Alternativ kann vorgesehen werden, daß sich der Innenumfang des Klappenringes in Blutströmungsrichtung stetig erweitert. Am Außenumfang des Klappenringes kann eine umlaufende Nut ausgespart sein, in welche ein Nahtring bekannter Konfiguration eingelegt ist, mit dessen Hilfe die Kunstklappe am Herzgewebe befestigt wird. Typischerweise besteht der Nahtring aus einem in die Nut am Klappenring eingelegten Kunststoff-Faden oder -Band, vorzugsweise aus "Dacron" (Wz), und einem über die Nut hinausstehenden Gewebe aus Kunststoff, vorzugsweise aus "Teflon"-Fasern (Wz). Vorzugsweise kann ein solcher Nahtring mit einer Orientierungsmarke versehen sein, welche dem Chirurgen ohne weiteres einen Hinweis auf den flächenmäßig größeren Klappenflügel sowie den flächenmäßig kleineren Klappenflügel vermittelt. Diese Orientierungsmarke kann beispielsweise in einer bestimmten Färbung, in einer entfernbaren Fahne oder dergleichen bestehen.

Vorzugsweise ist zusätzlich vorgesehen, daß der Klappenring innerhalb des am Gewebe befestigten Nahtringes drehbar gehalten ist. Diese drehbare Anordnung innerhalb des Nahtringes dient zur Feinjustierung der Prothese, nachdem der Nahtring am Gewebe festgelegt ist. Zweckmäßigerweise ist ein gewisser Kraftaufwand für die Verdrehung vorgesehen, um eine unbeabsichtigte Verstellung des Klappenringes zu verhindern.

Nachstehend wird die erfindungsgemäße Doppelflügel-Herzklappenprothese mehr im einzelnen anhand bevorzugter Ausführungsformen mit Bezugnahme auf die Zeichnungen erläutert; die letzteren zeigen:
- Fig. 1: in einer vereinfachten schematischen Darstellung die linken Herzräume im a.t. Strahlengang (Röntgenbild), wobei mit dicken ausgezogenen Linien die Taschenklappen des natürlichen Aortenklappenapparates angedeutet sind;
- Fig. 2: eine Darstellung analog zu Fig. 1, jedoch mit einer erfindungsgemäßen Prothese in der Aortenposition;
- Fig. 3: in einer vereinfachten schematischen Darstellung die linken Herzräume im seitlichen Strahlengang (Röntgenbild), wobei zusätzlich der Klappenring und die beiden Hauptsegel des natürlichen Mitralklappenapparates angedeutet sind;
- Fig. 4: eine Darstellung analog zu Fig. 3, jedoch mit einer erfindungsgemäßen Prothese in der Mitralposition;
- Fig. 5: eine schematische Schrägansicht einer erfindungsgemäßen Doppelflügel-Herzklappenprothese zur Erläuterung von Definitionen;
- Fig. 6a: eine geschnittene Seitenansicht einer ersten Ausführungsform einer erfindungsgemäßen Prothese, wobei die Klappenflügel eine Offen-Stellung einnehmen;
- Fig. 6b: eine Darstellung analog zu Fig. 6a, wobei jedoch die Klappenflügel eine Schließ-Stellung einnehmen;
- Fig. 6c: eine Draufsicht von unten (in Strömungsrichtung) auf die Prothese nach Fig. 6b;
- Fig. 7a: eine Seitenansicht und
- Fig. 7b: eine Draufsicht der Prothese nach Fig. 6a und 6b;
- Fig. 8a: eine Seitenansicht und
- Fig. 8b: eine Draufsicht einer abgeänderten Prothese nach Fig. 6a und 6b;
- Fig. 9a: eine geschnittene Seitenansicht und
- Fig. 9b: eine weitere geschnittene Seitenansicht und
- Fig. 9c: eine Draufsicht einer zweiten Ausführungsform einer erfindungsgemäßen Prothese mit ausgedehnten, länglichen, schräg gestellten Achslagern.

Die Zeichnungen dienen lediglich zur Erläuterung der Erfindung, ohne diese einzuschränken, und zeigen die Kunstklappe größer als in der Praxis realisiert.

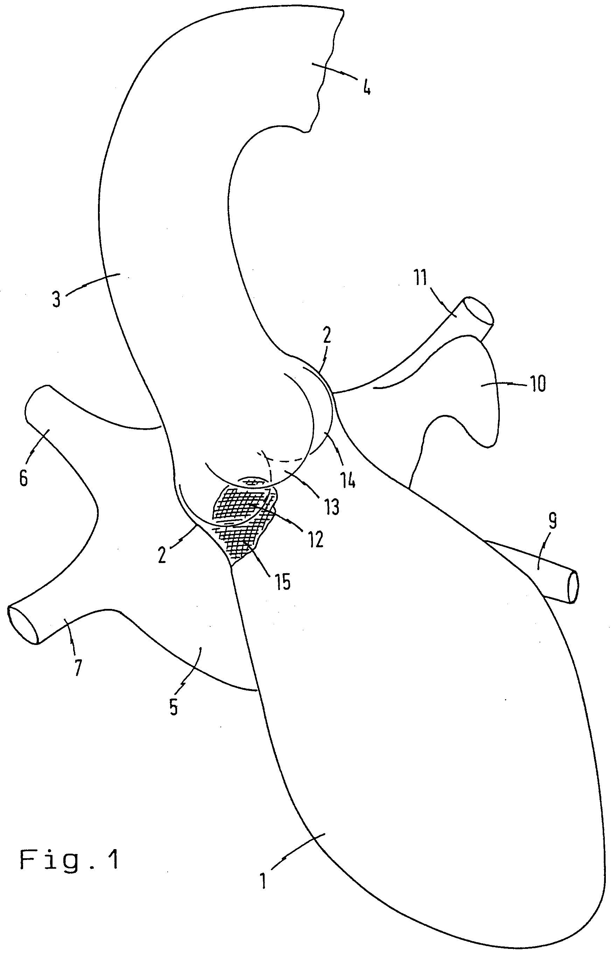

Die Fig. 1 zeigt in einer vereinfachten, schematischen Darstellung die linken Herzräume im a.t. Strahlengang (Röntgenbild) nach Frank H. Metter "Farbatlanten der Medizin, The Ciba Collection of Medical Illustrations", Band 1: Herz, Seite 28, 3. Aufl., 1990, Georg Thieme Verlag, Stuttgart-New York. Im einzelnen sind im Vordergrund zu erkennen: der linke Ventrikel 1, die Aortenbulben 2, die Aorta ascendens 3 und der Aortenbogen 4. Im Hintergrund befinden sich der linke Vorhof 5 mit V. pulmonalis sup. dext. 6, V. pulmonalis inf. dext. 7 und V. pulmonalis inf. sin. 9, sowie das linke Herzohr 10 mit V. pulmonalis sup. sin. 11.

Innerhalb der Aortenbulben 2 befinden sich die Segel oder Klappen des Aortenklappenapparates, nämlich die hintere Taschenklappe 12, die linke Taschenklappe 13 und die rechte Taschenklappe 14, die schematisch mit Halbkreisbögen angedeutet sind. Mit einer Schraffierung ist das membranöse Septum 15 angedeutet.

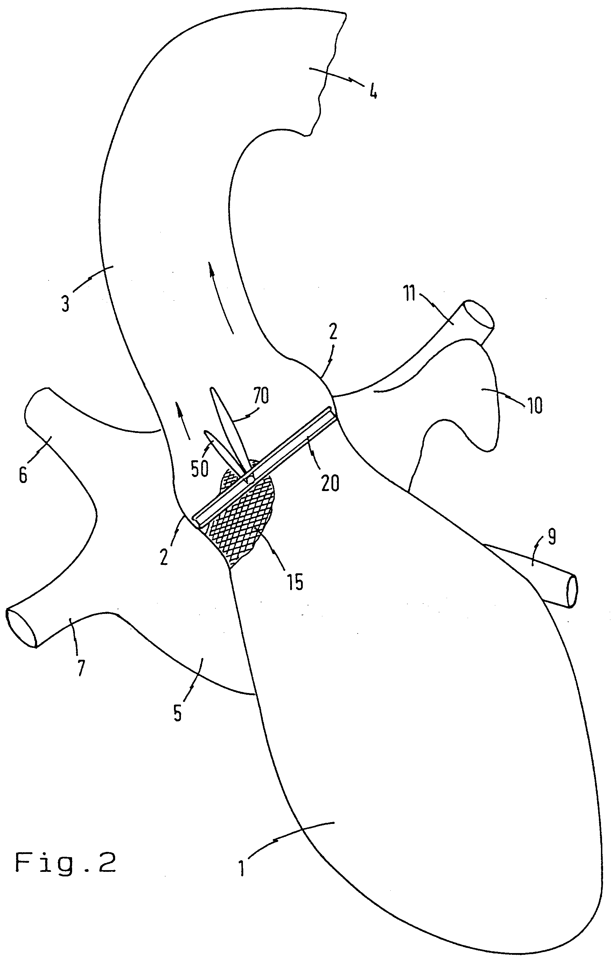

In der Darstellung nach Fig. 2 sind diese Taschenklappen des natürlichen Aortenklappenapparates durch eine erfindungsgemäße Kunstklappe ersetzt. Beim Aortenklappenersatz werden die erkrankten Klappensegel exzidiert; ferner werden Kalkspangen am Klappenring und an der Ausflußbahn des linken Ventrikels abgetragen. An der Aortenwand wird jedoch ein kleiner Klappenrest übrig gelassen, an welchem der Nahtring der Kunstklappe festgenäht wird. Hierbei ist besonders darauf zu achten, daß der Oberrand der Kunstklappe ein gutes Stück unterhalb der Koronarostien zu liegen kommt. Ferner muß eine Behinderung des Mitralklappensegels verhindert werden. Dies führt zu Beschränkungen beim Klappenersatz in der Aortenposition.

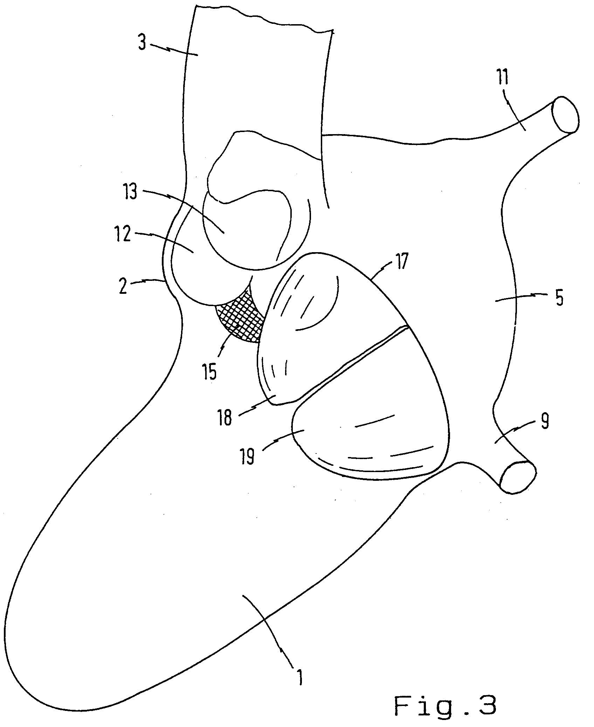

Die Fig. 3 zeigt in einer vereinfachten, schematischen Darstellung die linken Herzräume im seitlichen Strahlengang (Röntgenbild) nach Frank H. Metter "Farbatlanten der Medizin, The Ciba Collection of Medical Illustrations", Band 1: Herz, Seite 29, 3. Aufl., 1990, Georg Thieme Verlag, Stuttgart-New York, mit den oben bereits erläuterten Komponenten. Zusätzlich sind der linke Vorhof 5 und der Mitralklappenapparat mit Mitralklappenring 17, dem vorderen Segel 18 und dem hinteren Segel 19 angedeutet.

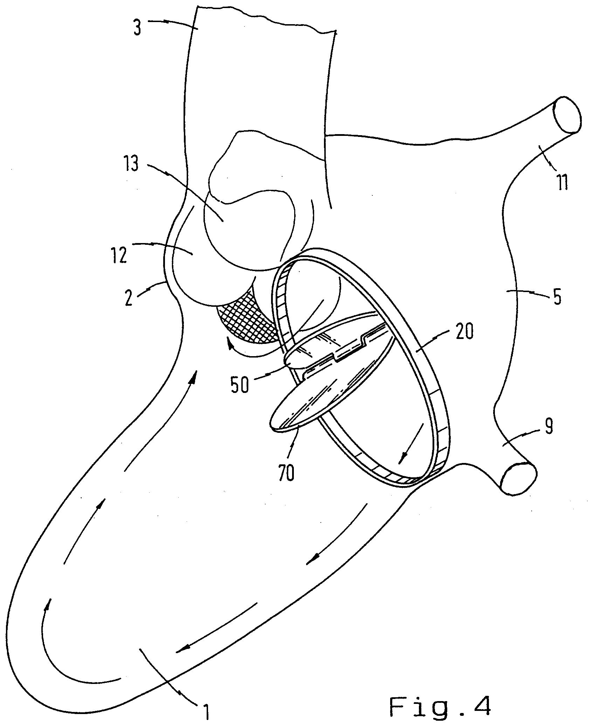

In der Darstellung nach Fig. 4 sind die Segel der Mitralklappe durch eine erfindungsgemäße Kunstklappe ersetzt. Beim Mitralklappenersatz werden zumeist die Papillarmuskeln durchtrennt und die Segel mit Sehnenfäden und Muskelstümpfen exzidiert. Der Nahtring der Kunstklappe wird am Mitralklappenring angenäht. Es besteht insbesondere die Gefahr einer Beschädigung oder Behinderung eines Aortenklappensegels. Ferner kann der beim Schließen der Aortenklappensegel erzeugte Blutstrom (Aorten-Regurgitation) das Schließen der Klappenflügel in der Mitralposition verzögern.

In den Darstellungen nach Fig. 2 und Fig. 4 nehmen die Klappenflügel eine Offen-Stellung ein, und es ist gut zu erkennen, daß die asymmetrische Anordnung der Klappenflügel in dieser Position offensichtlich ein Strömungsprofil erzeugt, das besser an die Anatomie und Physiologie des Herzens angepaßt ist als bei symmetrischer Anordnung der beiden Klappenflügel.

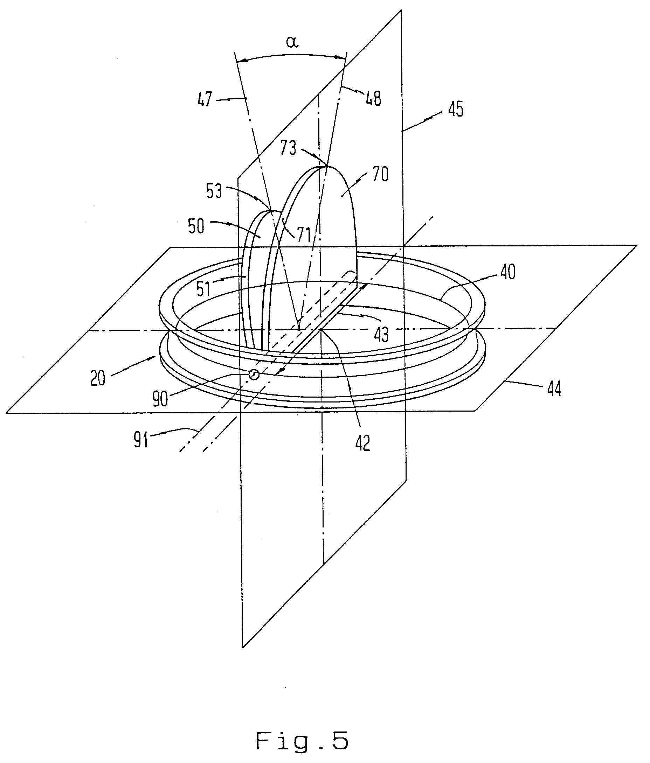

Mit Bezugnahme auf Fig. 5 werden verschiedene Definitionen erläutert, die im Rahmen der vorliegenden Unterlagen verwendet werden. Der Klappenring 20 definiert einen entsprechenden Kreis 40, der einen Mittelpunkt 42 aufweist. Der Kreis 40 erstreckt sich in und definiert damit eine Klappenringebene 44. Eine Normalmittelebene 45 zur Klappenringebene 44 erstreckt sich vertikal zur Klappenringebene 44 und schließt den Mittelpunkt 42 des Kreises 40 ein. Weiterhin weist der Kreis 40 einen Durchmesser 43 auf, der in der Normalmittelebene 45 liegt. Die erfindungsgemäße asymmetrische Anordnung der Klappenflügel 50, 70 ist dadurch gekennzeichnet, daß eine Längsmittelachse 91 der Achse 90, an welcher die beiden Klappenflügel 50, 70 angelenkt sind, einen Abstand zur Normalmittelebene 45 bzw. zum Durchmesser 43 aufweist. Mit anderen Worten ausgedrückt, die Achse 90 ist parallel und im Abstand zu einem Kreisdurchmesser 43 am Klappenring 20 ausgerichtet und ist insoweit exzentrisch bzw. asymmetrisch zur Normalmittelebene 45 zur Klappenringebene 44 angeordnet.

Ferner wird ein Klappenflügel-Öffnungswinkel (α) begrenzt durch zwei Gerade 47 und 48, welche die Längsmittelachse 91 schneiden und durch den Scheitel 53 oder 73 des einen Klappenflügels 50 oder des anderen Klappenflügels 70 führen.

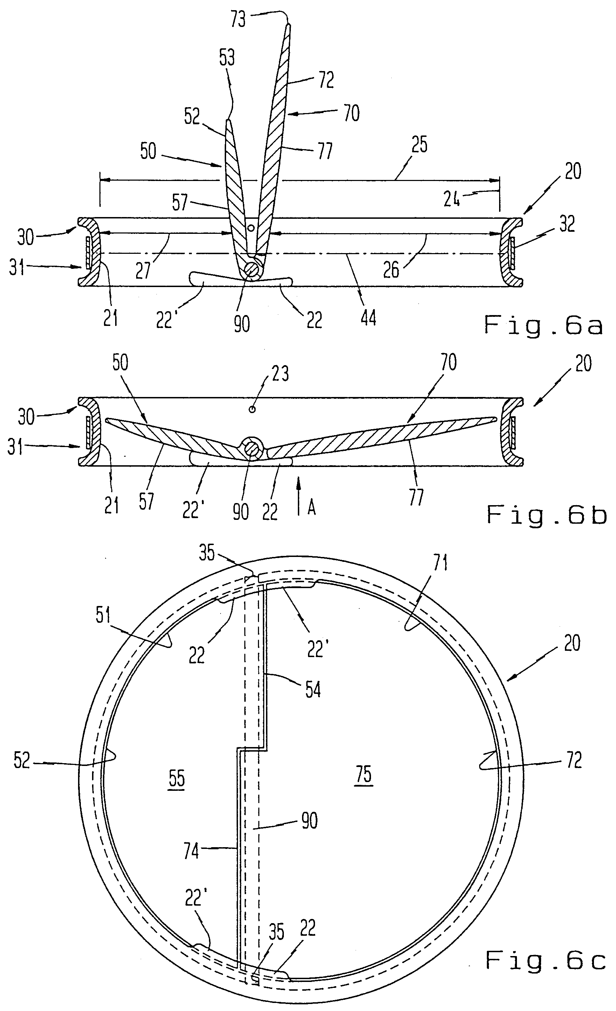

Zu den wesentlichen Bestandteilen der in den Figuren 6a bis 9c im einzelnen dargestellten Prothesen gehören ein Klappenring 20 und die beiden Klappenflügel 50 und 70 sowie eine Achse 90.

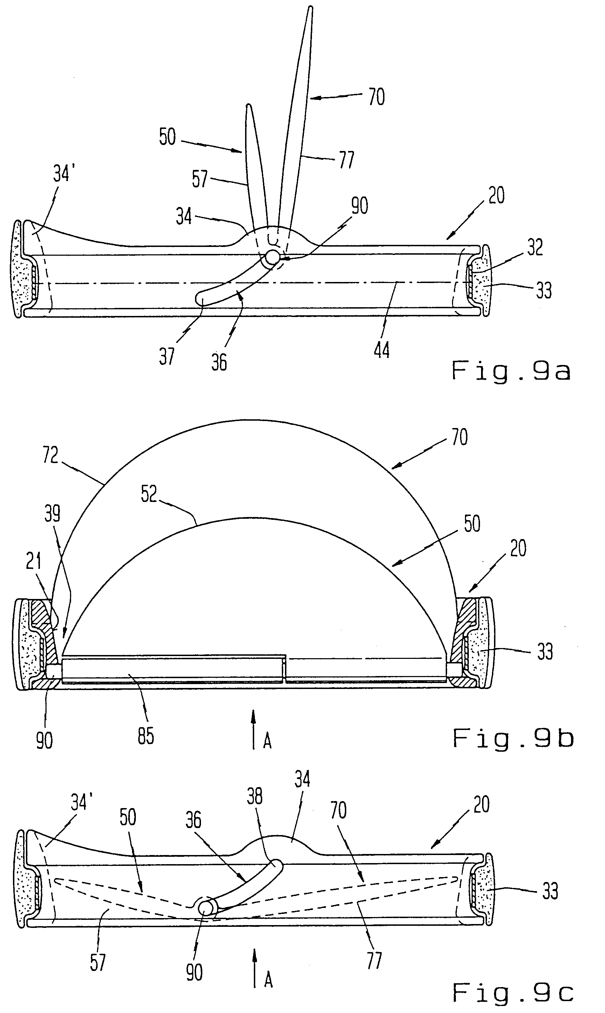

Der Klappenring 20 ist typischerweise kreisrund ausgebildet und weist im Regelfall eine gleichbleibende Höhe auf. Alternativ kann der Klappenring 20 auf einer Seite, benachbart zum flächenmäßig kleineren Klappenflügel 50, eine größere Höhe aufweisen (Klappenringabschnitt 34', vgl. Fig. 9a, 9c). Weiterhin kann der Klappenring 20 im Bereich der Ohren 34 (vgl. Fig. 9a, 9c) eine größere Höhe aufweisen, um ausreichenden Platz für ausgedehnte, längliche, schräg gestellte Achslager 36 zu schaffen. Wie aus Fig. 6a ersichtlich, weist der Klappenring 20 einen Innenumfang 21 auf, der einen Strömungskanal 24 für die Blutströmung begrenzt. Diejenige Richtung der Blutströmung, welche die Klappenflügel 50, 70 aus der Schließ-Stellung in die Offen-Stellung verstellt, ist mit den Pfeilen A angedeutet. Der Strömungskanal 24 weist einen Strömungskanalquerschnitt 25 auf, der sich in der Klappenringebene 44 erstreckt. Entsprechend der asymmetrischen Anordnung der Klappenflügel 50, 70 weist der Strömungskanalquerschnitt 25 einen größeren Abschnitt 26 und einen kleineren Abschnitt 27 auf. In der Aortenposition befindet sich der größere Strömungskanalquerschnittsabschnitt 26 auf der dorsalen Seite, und der kleinere Strömungskanalquerschnittsabschnitt 27 befindet sich auf der ventralen Seite. In der Mitralposition befindet sich der größere Strömungskanalquerschnittsabschnitt 26 auf der dorsalen Seite, und der kleinere Strömungskanalquerschnittsabschnitt 27 befindet sich auf der ventralen Seite. Der Innenumfang 21 weist vorzugsweise eine konvexe Wölbung auf.

Ferner hat der Klappenring 20 einen Außenumfang 30, an dem typischerweise eine umlaufende Nut 31 ausgespart ist, in welche ein Band 32 oder Faden aus Kunststoff (vorzugsweise "Dacron" (Wz)) eingelegt ist, an dem in bekannter Weise ein Nahtring befestigt wird, mit dessen Hilfe die Kunstklappe am Gewebe befestigt wird. Ein solcher Nahtring 33 ist beispielsweise in den Figuren 9a, 9b, 9c schematisch angedeutet.

Jeder Klappenflügel 50, 70 weist einen im wesentlichen halbmondförmigen Umfang oder Umriß 51, 71 auf, der von einem bogen- bzw. halbkreisförmigen Randabschnitt 52, 72 und von einem geraden Randabschnitt 54, 74 gebildet wird. Entfernt (distal) zum geraden Randabschnitt 54, 74 weist jeder bogenförmige Randabschnitt 52, 72 einen Scheitel 53, 73 auf. Der Umriß 51, 71 begrenzt eine Fläche 55, 75 jedes Klappenflügels 50, 70 im Sinne einer Querschnittsfläche in der Ebene des Klappenflügels.

Bei im wesentlichen benachbarter, V-förmiger Anordnung weist jeder Klappenflügel 50, 70 eine innenliegende (proximale) Innenseite 56, 76 sowie eine außenliegende (distale) Außenseite auf, die nachstehend als Anströmseite 57, 77 bezeichnet wird. Die Innenseite 56, 76 und die Anströmseite 57, 77 begrenzen je ein Querschnittsprofil des Klappenflügels 50, 70, das durch eine sogenannte Skelettlinie beschrieben werden kann. Im Rahmen der vorliegenden Erfindung ist das Querschnittsprofil der Klappenflügel 50, 70 nicht besonders begrenzt und kann übereinstimmend oder unabhängig voneinander an der Innenseite 56, 76 und der Anströmseite 57, 77 eine gerade, ebene Konfiguration oder eine konvexe Konfiguration oder eine konkave Konfiguration oder in Richtung vom Scheitel 53, 73 bis zum geraden Randabschnitt 54, 74 eine zusammengesetzte Konfiguration aus verschiedenen Formen aufweisen. Solche zusammengesetzten Konfigurationen für die Innenseite 56, 76 und die Anströmseite 57, 77 der Klappenflügel 50, 70 werden beispielsweise in den Dokumenten WO 90/08519 oder EP 0 277 527 A1 beschrieben. Im Rahmen der vorliegenden Erfindung ist es auch möglich, an der Anströmseite 77 oder Innenseite 76 des flächenmäßig größeren Klappenflügels 70 eine andere Konfiguration vorzusehen als an der Anströmseite 57 oder Innenseite 56 des flächenmäßig kleineren Klappenflügels 50. Beispielsweise können am flächenmäßig kleineren Klappenflügel 50 die Unterschiede zwischen den verschiedenen Konfigurationsformen weniger ausgeprägt sein.

Die vorstehend erläuterten Klappenflügel 50, 70 sind schwenkbar in der Weise angeordnet, daß jeder Klappenflügel unabhängig vom anderen Klappenflügel allein unter der Wirkung von Druck und Richtung des anströmenden Blutes seine vorgesehene Position in der Offen-Stellung oder Schließ-Stellung einnehmen kann. Die schwenkbare Befestigung der Klappenflügel 50, 70 am Klappenring 20 erfolgt mittelbar über eine Achse 90, an der die beiden Klappenflügel 50, 70 angelenkt sind. Diese Achse 90 ist am Klappenring 20 drehbar oder ortsfest gehalten.

Mit den Figuren 6a, 6b und 6c ist eine erste Ausführungsform einer erfindungsgemäßen Prothese dargestellt. Der Klappenflügel 70 weist eine größere Flächengröße auf als der Klappenflügel 50. Die beiden Klappenflügel 50 und 70 sind - unabhängig voneinander schwenkbar - an der gemeinsamen Achse 90 angelenkt, die in Aussparungen 35 am Klappenring 20 gehalten ist. Diese Achse 90 weist eine Längsmittelachse 91 auf, die exzentrisch, d.h. parallel und im Abstand zu einem Durchmesser 43 an den jenigen Kreis 40 angeordnet ist, der durch den Klappenring 20 definiert wird (vgl. Fig. 5). Am Innenumfang 21 des Klappenringes 20 sind sanft und abgerundet mehrere Stufenabschnitte 22, 22' ausgebildet, auf welchen in der Schließstellung der bogenförmige Randabschnitt 52, 72 der Klappenflügel 50, 70 aufliegen kann. Wie dargestellt, erstreckt sich je ein Stufenabschnitt 22 oder 22' auf beiden Seiten des Achslagers 35 am Innenumfang 21 des Klappenringes 20. Weiterhin sind am Innenumfang 21 Vorsprünge 23 angeformt, welche die maximale Verstellung der Klappenflügel 50, 70 in deren Offen-Stellung begrenzen. In diesem Falle weist jeder Klappenflügel 50, 70 eine konvexe Konfiguration an der Anströmseite 57, 77 auf.

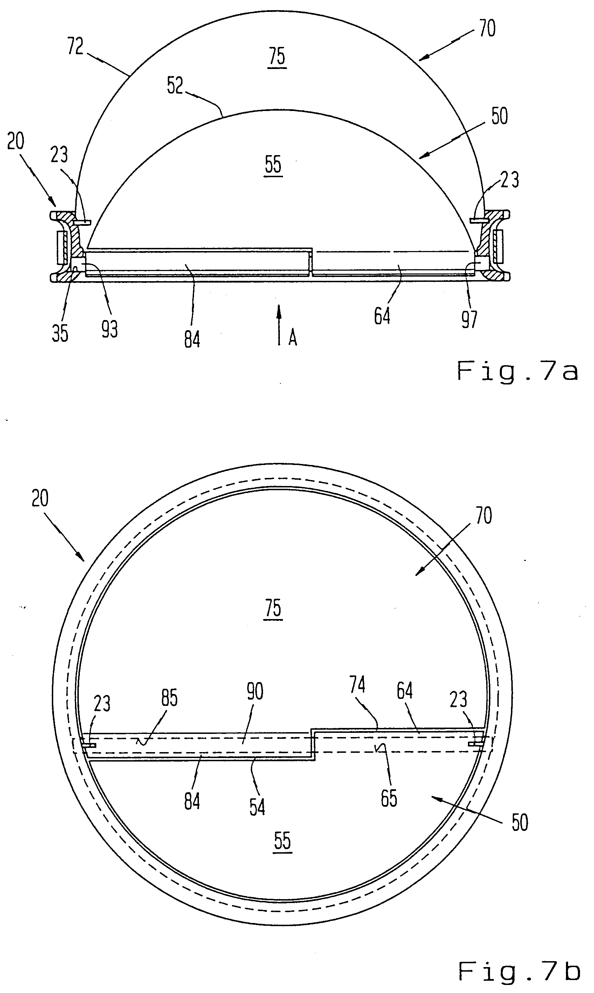

Mit den Figuren 7a und 7b ist für die Prothese nach den Figuren 6a bis 6c eine erste Ausgestaltung zur schwenkbaren Anordnung der Klappenflügel 50, 70 an der Achse 90 dargestellt. In diesem Falle ist am geraden Randabschnitt 54, 74 jedes Klappenflügels 50, 70 einstückig ein Hülsenabschnitt 64, 84 angeformt. Jeder Hülsenabschnitt 64, 84 beginnt am bogenförmigen Randabschnitt 52, 72 und erstreckt sich etwa bis zur Klappenflügelmitte. Anschließend weist die Klappenflügelfläche eine Aussparung auf, um Platz für den Hülsenabschnitt an dem anderen Klappenflügel zu schaffen. Am flächenmäßig größeren Klappenflügel 70 kann der Hülsenabschnitt 84 eine größere Länge aufweisen als der Hülsenabschnitt 64 am flächenmäßig kleineren Klappenflügel 50. Jeder Hülsenabschnitt 64, 84 weist eine durchgehende Bohrung 65, 85 auf. Bei passender, gegenüberstehender Anordnung der beiden Klappenflügel 50, 70 fluchten diese beiden Bohrungen 65, 85 miteinander, und durch die fluchtenden Bohrungen 65, 85 ist die Achse 90 geführt. Die Achse 90 weist gegenüberliegende Endabschnitte 93 und 97 auf, welche über die fluchtenden Bohrungen 65, 85 vorstehen und in je einem Achslager 35 am Klappenring 20 gehalten sind. In diesem Falle sind die Achslager 35 als durchgehende runde Bohrungen im Klappenring 20 ausgebildet.

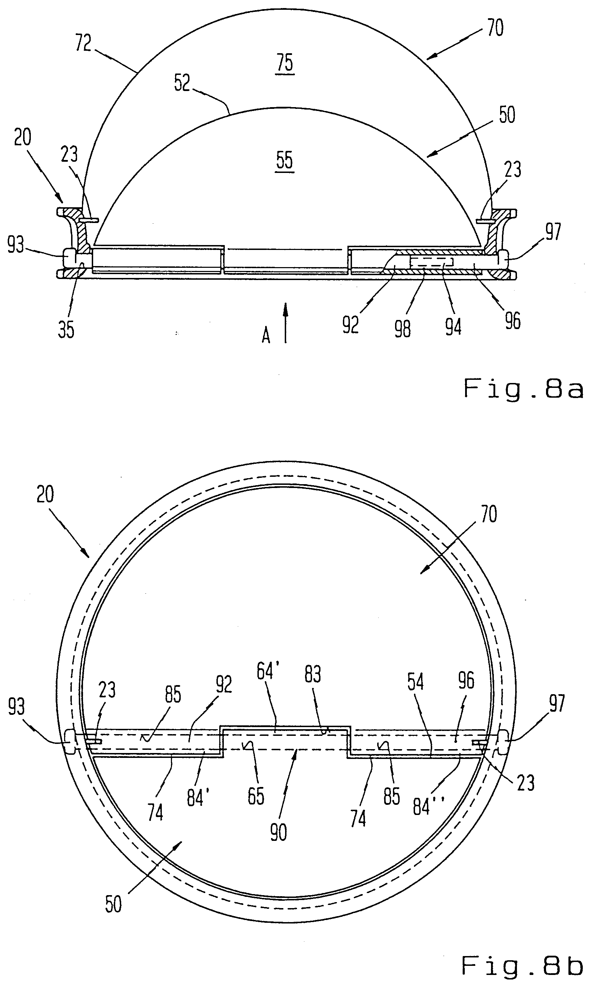

Mit den Figuren 8a und 8b ist für die Prothese nach den Figuren 6a bis 6c eine zweite Ausgestaltung zur schwenkbaren Befestigung der Klappenflügel 50, 70 an der Achse 90 dargestellt. In diesem Falle weist der flächenmäßig größere Klappenflügel 70 am geraden Randabschnitt 74 zwei einstückig angeformte Hülsenabschnitte 84', 84'' auf. Diese Hülsenabschnitte 84', 84'' erstrecken sich jeweils vom bogenförmigen Randabschnitt 72 nach innen und lassen eine Lücke 83 frei. In diese Lücke 83 ragt ein passender Hülsenabschnitt 64' hinein, der am geraden Randabschnitt 54 des flächenmäßig kleineren Klappenflügels 50 angeformt ist. Jeder Hülsenabschnitt 64', 84', 84'' weist eine durchgehende Bohrung 65, 85 auf, und bei passender, gegenüberstehender Anordnung der Klappenflügel 50, 70 fluchten diese Bohrungen 65, 85. Durch die fluchtenden Bohrungen 65, 85 ist die Achse 90 geführt.

In diesem Falle ist die Achse 90 zweiteilig ausgebildet und besteht aus den Achsenabschnitten 92 und 96. Jeder Achsenabschnitt 92, 96 weist an einem Ende einen verdickten Kopf 93, 97 auf. Das andere Ende jedes Achsenabschnittes 91, 95 ist als eine ineinandersteckbare Anordnung von Stift 94 und Hülse 98 ausgebildet. Jeder verdickte Kopf 93, 97 ist in einer passenden Aussparung 35 am Klappenring 20 gelagert.

Mit den Figuren 9a, 9b und 9c ist eine zweite Ausführungsform einer erfindungsgemäßen Prothese dargestellt. Wiederum sind die beiden Klappenflügel 50, 70 schwenkbar an einer gemeinsamen Achse 90 gelagert, deren Endabschnitte 93, 97 in je einem Achslager gehalten sind. In diesem Falle ist jedes Achslager als längliche, bezüglich der Klappenringebene 44 schräg gestellte Aussparung 36 ausgeführt. Wie dargestellt, erstreckt sich diese längliche Lageraussparung 36 von links unterhalb der Klappenringebene 44 nach rechts oberhalb der Klappenringebene 44. Bei dieser Anordnung weist das längliche Achslager 36 einen unteren Endabschnitt 37 und einen oberen Endabschnitt 38 auf. Es sind solche gegenseitigen Abmessungen vorgesehen, daß die Endabschnitte 93, 97 der Achse 90 beweglich, d.h. "schwimmend" in diesem länglichen Achslager 36 gehalten sind. In der Schließ-Stellung wird die Achse 90 eine Position im unteren Endabschnitt 37 einnehmen. Unter dem Druck des anströmenden Blutes erfolgt die Verschwenkung der Klappenflügel 50, 70 in deren Offen-Stellung. Gleichzeitig kann die Achse 90 in den oberen Abschnitt 38 des länglichen Achslagers 36 wandern. Damit kann der Druckgradient vermindert und der Energieverlust gesenkt werden.

Wie dargestellt, ist der Klappenring 20 im Bereich der ausgedehnten, länglichen, schräg gestellten Achslager 36 mit sogenannten Ohren 34 versehen, die eine größere Höhe aufweisen.

Wie aus Fig. 9b ersichtlich, weist der Klappenring 20 in diesem Falle an seinem Innenumfang 21 eine Kontur oder Konfiguration auf, die sich in Blutströmungsrichtung (Pfeil "A") stetig erweitert. Sofern sich die Achse 90 im unteren Endabschnitt 37 des länglichen Achslagers 36 befindet, besteht eine gewisse Spaltbreite 39 zwischen der Stirnfläche des Hülsenabschnittes 85 mit dem anschließenden bogenförmigen Randabschnitt 72 zum Innenumfang 21 des Klappenringes 20. Bei der Wanderung der Achse 90 in den oberen Endabschnitt 38 des länglichen Achslagers 36 im Verlauf des Öffnungsvorganges nimmt diese Spaltbreite 39 zu. Hierdurch wird die Spülung dieser Lagerzone mit frischem Blut verbessert.

Zusätzlich kann - wie dargestellt - für den Klappenring 20 benachbart zu dem flächenmäßig kleineren Klappenflügel 50 ein Klappenringabschnitt 34' vorgesehen werden, der eine größere Höhe aufweist.

## Patentansprüche

1. Doppelflügel-Herzklappenprothese
mit einem im wesentlichen kreisrunden Klappenring (20), dessen Innenumfang (21) einen Strömungskanal (24) für die Blutströmung begrenzt, und
mit zwei im wesentlichen halbmondförmigen Klappenflügeln (50, 70), die beide und unabhängig voneinander schwenkbar an einer gemeinsamen Achse (90) angelenkt sind, die am klappenring (20) gehalten ist, wobei beide Klappenflügel (50,70) - abhängig von der Blutströmungsrichtung - eine Offen-Stellung im wesentlichen vertikal zur Klappenringebene (44) oder eine Schließ-Stellung im wesentlichen in Richtung der Klappenringebene (44) einnehmen können, in welcher beide Klappenflügel (50, 70) mit ihren zusammengenommenen Flächen (55, 75) den Strömungskanal (24) nahezu vollständig verschließen,
dadurch gekennzeichnet, daß
die Achse (90) exzentrisch, das heißt parallel und im Abstand zu einem Kreisdurchmesser (43) am Klappenring (20) angeordnet ist; und
der eine Klappenflügel (70) eine größere Fläche (75) aufweist als der andere Klappenflügel (50).

2. Doppelflügel-Herzklappenprothese nach Anspruch 1,
dadurch gekennzeichnet, daß
die Exzentrizität der Achse (90), das heißt der Abstand einer Längsmittelachse (91) der Achse (90) zu einem Durchmesser (43) am Klappenring (20) etwa 4 bis 22 % der Länge eines Innendurchmessers des Klappenringes (20) ausmacht.

3. Doppelflügel-Herzklappenprothese nach Anspruch 1 oder 2,
dadurch gekennzeichnet, daß
der Strömungskanal (24) einen Strömungskanalquerschnitt (25) aufweist; und
der flächenmäßig größere Klappenflügel (70) etwa 55 bis 80 % des Strömungskanalquerschnittes (25) verschließt; und
der flächenmäßig kleinere Klappenflügel (50) etwa 45 bis 20 % des Strömungskanalquerschnittes (25) verschließt.

4. Doppelflügel-Herzklappenprothese nach Anspruch 3,
dadurch gekennzeichnet, daß
der flächenmäßig größere Klappenflügel (70) etwa 60 bis 68 % des Strömungskanalquerschnittes (25) verschließt; und
der flächenmäßig kleinere Klappenflügel (50) etwa 40 bis 32 % des Strömungskanalquerschnittes (25) verschließt.

5. Doppelflügel-Herzklappenprothese nach einem der Ansprüche 1 bis 4
dadurch gekennzeichnet, daß
für die Anwendung als Aortenprothese der flächenmäßig größere Klappenflügel (70) dorsal angeordnet wird.

6. Doppelflügel-Herzklappenprothese nach einem der Ansprüche 1 bis 4
dadurch gekennzeichnet, daß
für die Anwendung als Mitralprothese der flächenmäßig größere Klappenflügel (70) dorsal angeordnet wird.

7. Doppelflügel-Herzklappenprothese nach einem der Ansprüche 1 bis 6,
dadurch gekennzeichnet, daß
die Achse (90) zwei Endabschnitte (93, 97) aufweist; und
jeder Achsen-Endabschnitt (93, 97) in einem Achslager gehalten ist, das als Aussparung im Klappenring (20) ausgebildet ist; und
jedes Achslager als längliche Aussparung (36) ausgebildet ist, die sich in einer Richtung von links unterhalb nach rechts oberhalb bezüglich der Klappenringebene (44) erstreckt.

8. Doppelflügel-Herzklappenprothese nach Anspruch 7,
dadurch gekennzeichnet, daß
der Innenumfang (21) des Klappenringes (20) zumindest im Bereich der beiden Achslager (36) in Blutströmungsrichtung zunimmt.

9. Doppelflügel-Herzklappenprothese nach Anspruch 7 oder 8,
dadurch gekennzeichnet, daß
der Klappenring (20) im Bereich der Achslager (36) eine größere Höhe und/oder eine Ausweitung aufweist.

10. Doppelflügel-Herzklappenprothese nach einem der Ansprüche 1 bis 9,
dadurch gekennzeichnet, daß
die Achse (90) zweiteilig ausgebildet ist und aus zwei Achsenabschnitten (92, 96) besteht;
jeder Achsenabschnitt (92, 96) an einem Ende einen verdickten Kopf (93, 97) aufweist, der im Achslager (35, 36) gehalten ist; und
die beiden anderen Endabschnitte der Achsenabschnitte (92, 96) als Stift (98) und Hülse (99) ausgebildet sind, die in-einander steckbar sind.

11. Doppelflügel-Herzklappenprothese nach einem der Ansprüche 1 bis 10,
dadurch gekennzeichnet, daß
jeder Klappenflügel (50, 70) halbmondförmig ausgebildet ist mit einem bogenförmigen Randabschnitt (52, 72) und mit einem geraden Randabschnitt (54, 74);
am geraden Randabschnitt (54, 74) wenigstens ein Hülsenabschnitt (64, 84) einstückig angeformt ist, durch dessen Bohrung (65, 85) die Achse (90) geführt ist;
der Außendurchmesser der Achse (90) und der Innendurchmesser der Bohrung (65, 85) der Hülsenaschnitte (64, 84) derartig bemessen sind, daß einerseits eine leichtgängige Verschwenkbarkeit der Klappenflügel (50, 70) um die Achse (90) gewährleistet ist, und daß andererseits Blut im wesentlichen nicht in die Bohrung (65, 85) der Hülsenabschnitte (64, 84) eindringen kann.

12. Doppelflügel-Herzklappenprothese nach einem der Ansprüche 1 bis 11,
dadurch gekennzeichnet, daß
jeder Klappenflügel (50, 70) halbmondförmig ausgebildet ist, mit einem bogenförmigen Randabschnitt (52, 72) und mit einem geraden Randabschnitt (54, 74),
am geraden Randabschnitt (54, 74) wenigstens ein Hülsenabschnitt (64, 84) einstückig angeformt ist, durch dessen Bohrung (65, 85) die Achse (90) geführt ist; und
jeder Klappenflügel (50, 70) auf seiner Anströmseite (57, 77) vom Scheitel (53, 73) des bogenförmigen Randabschnittes (52, 72) bis zum geraden Randabschnitt (54, 74) eine konvexe Konfiguration aufweist, die stetig in die Krümmung des Hülsenabschnittes (64,84) übergeht.

13. Doppelflügel-Herzklappenprothese nach einem der Ansprüche 1 bis 12,
dadurch gekennzeichnet, daß
die beiden Klappenflügel (50, 70) in der Offen-Stellung einen Klappenflügel-Öffnungswinkel (α) von etwa 10 bis 40° einschließen, der durch je eine Gerade (47, 48) begrenzt ist, die durch die Längsmittelachse (91) der Achse (90) und den Scheitel (53, 73) am bogenförmigen Randabschnitt (52, 72) jedes Klappenflügels (50, 70) führt.

14. Doppelflügel-Herzklappenprothese nach Anspruch 13,
dadurch gekennzeichnet, daß
die beiden Klappenflügel (50, 70) in der Offen-Stellung einen Klappenflügel-Öffnungswinkel (α) von etwa 12 bis 18° einschließen.

15. Doppelflügel-Herzklappenprothese nach einem der Ansprüche 1 bis 14,
dadurch gekennzeichnet, daß
die Klappenflügel (50, 70) in der Offen-Stellung eine Position einnehmen, die im wesentlichen der Richtung der Strömungslinien der Blutströmung entspricht.

16. Doppelflügel-Herzklappenprothese nach einem der Ansprüche 1 bis 15,
dadurch gekennzeichnet, daß
am Innenumfang (21) des Klappenringes (20) Anschläge ausgebildet sind, die ein Hinausschwingen der Klappenflügel (50, 70) über deren vorgesehene Position in der Schließ-Stellung verhindern; und
diese Anschläge als je zwei Stufenabschnitte (22, 22') ausgebildet sind, die sich auf beiden Seiten eines Achslagers (35, 36) über eine relativ kurze Strecke am Innenumfang (21) im wesentlichen im Richtung der Klappenringebene (44) erstrecken.

17. Doppelflügel-Herzklappenprothese nach Anspruch 16,
dadurch gekennzeichnet, daß
jeder Stufenabschnitt (22, 22') eine Länge von etwa 3 bis 6 mm aufweist.

18. Doppelflügel-Herzklappenprothese nach Anspruch 16 oder 17,
dadurch gekennzeichnet, daß
sich jeder Stufenabschnitt (22, 22') in einer Richtung erstreckt, die vom Achslager (35) zum - in Strömungsrichtung - oberen (distalen) Rand des Klappenringes (20) führt.

19. Doppelflügel-Herzklappenprothese nach einem der Ansprüche 1 bis 18,
dadurch gekennzeichnet, daß
am Innenumfang (21) des Klappenringes (20) Anschläge ausgebildet sind, die ein Hinausschwingen der Klappenflügel (50, 70) über deren vorgesehene Position in der Offen-Stellung verhindern; und
diese Anschläge als zwei Stifte (23) ausgebildet sind, die im - in Strömungsrichtung - oberen (distalen) Bereich des Klappenringes (20) vom Innenumfang (21) nach innen vorstehen.

20. Doppelflügel-Herzklappenprothese nach einem der Ansprüche 1 bis 19,
dadurch gekennzeichnet, daß
der Klappenring (20) wenigstens einen Klappenringabschnitt (34') mit einer größeren Höhe aufweist; und
dieser Klappenringabschnitt (34') benachbart zu dem flächenmäßig kleineren Klappenflügel (50) angeordnet ist.

21. Doppelflügel-Herzklappenprothese nach einem der Ansprüche 1 bis 20,
dadurch gekennzeichnet, daß
der Klappenring (20) und/oder die Klappenflügel (50, 70) einen mehrschichtigen Aufbau aufweisen, mit einer Deckschicht aus pyrrolytischem Kohlenstoff, mit einer oder mehreren Zwischenschichten aus Titancarbid(en) und mit einem Kern aus metallischem Titan.

## Claims

1. A bi-leaflet prosthetic heart valve
comprising
- an essentially circular valve ring (20) having an inner circumference (21) defining a flow channel (24) for the blood flow;
- an axle (90) fastened to said valve ring (20);
- two essentially half-moon-shaped leaflets (50, 70), both of which being hinged for pivoting independently of each other on said common axle (90), and being enabled - depending on the direction of the blood flow - to assume an open position, wherein both leaflets (50, 70) extend essentially at right angles to a valve ring plane (44) and to assume a closed position, wherein both leaflets (50, 70) extend essentially in the direction of the valve ring plane (44), and virtually occlude the flow channel (24) with their combined areas (55, 75);
characterized in that
said axle (90) being arranged to said valve ring (20) excentrically, that is parallel and distantly to a diameter (43) of a circle (40) defined by the valve ring (20); and
one leaflet (70) comprises a greater area (75) than the area (55) of the other leaflet (50).

2. The bi-leaflet prosthetic heart valve according to claim 1,
wherein
the axle (90) comprises an excentricity, that is a distance between a longitudinal central axis (91) of the axle (90) and a diameter (43) of the circle (40) defined by the valve ring (20); and wherein said excentricity amounts about 4 % to 22 % of the length of said inner diameter (43) of the circle (40) defined by the valve ring (20).

3. The bi-leaflet prosthetic heart valve according to claim 1 or 2,
wherein
the flow channel (24) comprises a flow channel cross-section (25); and
wherein the leaflet (70) of larger area occludes about 55 % to 80 % of the flow channel cross-section (25); and
wherein the leaflet (50) of smaller area occludes about 45 % to 20 % of the flow channel cross-section (25).

4. The bi-leaflet prosthetic heart valve according to claim 3,
wherein
the leaflet (70) of larger area occludes about 60 % to 68 % of the flow channel cross-section (25); and
wherein the leaflet (50) of smaller area occludes about 40 % to 32 % of the flow channel cross-section (25).

5. The bi-leaflet prosthetic heart valve according to anyone of the claims 1 to 4,
intended for use as an aortal prosthesis,
wherein the leaflet (70) of larger area being arranged dorsal.

6. The bi-leaflet prosthetic heart valve according to anyone of the claims 1 to 4,
intended for use as a mitral prosthesis,
wherein the leaflet (70) of larger area being arranged dorsal.

7. The bi-leaflet prosthetic heart valve according to anyone of the claims 1 to 6,
wherein
the axle (90) comprises two end portions (93, 97); and
each end portion (93, 97) being retained in a shaft bearing formed like a recess in the valve ring (20); and
each shaft bearing being formed like a longitudinal recess (36) extending from the left side below the valve ring plane (44) to the right side above the valve ring plane (44).

8. The bi-leaflet prosthetic heart valve according to claim 7,
wherein
the valve ring (20) comprises an inner circumference (21); and
said inner circumference (21) comprises a configuration or contour which widens at least in the area of the two shaft bearings (36) in the direction of the blood flow.

9. The bi-leaflet prosthetic heart valve according to claim 7,
wherein
the valve ring (20) comprises a greater height and/or a larger wide in the area of the shaft bearings (36).

10. The bi-leaflet prosthetic heart valve according to anyone of the claims 1 to 9,
wherein
the axle (90) comprises a two-part structure consisting of two axle portions (92, 96);
wherein each axle portion (92, 96) comprises at one end a thickened head portion (93, 97), retained within a shaft bearing (35, 36); and
comprises at the other end an opposite end portion, wherein one opposite end portion being formed like a socket (98), and the other opposite end portion being formed like a pin (94), enabled to be inserted in said socket (98).

11. The bi-leaflet prosthetic heart valve according to anyone of the claims 1 to 10,
wherein
each leaflet (50, 70) being shaped like a half-moon and being defined by a curved edge section (52, 72) and by a straight edge section (54, 74);
wherein each leaflet (50, 70) comprises adjacent to said straight edge section (54, 74) at least one integrally formed sleeve portion (64, 84) having a bore (65, 85);
wherein the axle (90) extending through said bores (65, 85); and wherein the external diameter of the axle (90) and the internal diameter of the bores (65, 85) comprise dimensions such that first the leaflets (50, 70) may pivot easily around the axle (90) and secondly, blood is essentially hindered to enter said bores (65, 85).

12. The bi-leaflet prosthetic heart valve according to anyone of the claims 1 to 11,
wherein
each leaflet (50, 70) being shaped like a half-moon and being defined by a curved edge section (52, 72) and by a straight edge section (54, 74);
wherein each leaflet (50, 70) comprises adjacent to said straight edge section (54, 74) at least one integrally formed sleeve portion (64, 84) having a bore (65, 85);
wherein the axle (90) extending through said bores (65, 85); and
wherein each leaflet (50, 70) comprises on a side (57, 77) facing the blood flow a convex configuration, extending from a crown point (53, 73) of the curved edge section (52, 72) to the straight edge section (54, 74) and comprising a continuous transition into the curvature of the sleeve portion (64, 84).

13. The bi-leaflet prosthetic heart valve according to anyone of the claims 1 to 12,
wherein
an arrangement of the leaflets (50, 70) being designated by a leaflet aperture angle (α) defined by two straight lines (47,48) passing through a longitudinal central axis (91) of the axle (90) and the crown point (53, 73) of the curved edge section (52, 72) of each leaflet (50, 70); and
wherein said leaflet aperture angle (α) amounts about 10° to 40° in an open position of the leaflets (50, 70).

14. The bi-leaflet prosthetic heart valve according to claim 13,
wherein
said leaflet aperture angle (α) amounts about 12° to 18° in an open position of the leaflets (50, 70).

15. The bi-leaflet prosthetic heart valve according to anyone of the claims 1 to 14,
wherein
the leaflets (50, 70) assume in the open position a position corresponding essentially to the orientation of the flow lines of the blood flow.

16. The bi-leaflet prosthetic heart valve according to anyone of the claims 1 to 15,
wherein
the inner circumference (21) of the valve ring (20) comprises stops preventing the leaflets (50, 70) from oscillating beyond their intended position in the closed position; and
said stops being formed like two step portions (22, 22') extending inwardly on both sides of a shaft bearing (35, 36) over a relatively short distance along the internal circumference (21) essentially in the direction of the valve ring plane (44).

17. The bi-leaflet prosthetic heart valve according to claim 16,
wherein
each step portion (22, 22') comprises a length of about 3 mm to 6 mm.

18. The bi-leaflet prosthetic heart valve according to claim 16 or 17,
wherein
each step portion (22, 22') extends in a direction extending from the shaft bearing (35) to the upper (distal) edge of the valve ring (20) in the flow direction.

19. The bi-leaflet prosthetic heart valve according to anyone of the claims 1 to 18,
wherein
the inner circumference (21) of the valve ring (20) comprises stops, preventing the leaflets (50, 70) from oscillating beyond their intended position in the open position; and
said stops being formed like two pins (23), extending inwardly from the internal circumference (21) within an upper (distal) area of the valve ring (20) in the flow direction.

20. The bi-leaflet prosthetic heart valve according to anyone of the claims 1 to 19,
wherein
the valve ring (20) comprises at least one valve ring section (34') having greater height; and
said section (34') being arranged adjacent to the leaflet (50) of smaller area.

21. The bi-leaflet prosthetic heart valve according to anyone of the claims 1 to 20,
wherein
the valve ring (20) and/or the leaflets (50, 70) comprise a multi-layered structure including a cover layer made of pyrolytic carbon, and further including one or more intermediate layers made of titanium carbide(s) and further comprising a core made of metallic titanium.

## Revendications

1. Prothèse de valve du coeur à aile double,
comportant un anneau (20) de valve sensiblement circulaire, dont la circonférence intérieure (21) délimite un canal (24) d'écoulement du sang,
et comportant deux ailes (50,70) de valve sensiblement semi-lunaires, qui sont articulées toutes deux et indépendamment l'une de l'autre de manière à pouvoir basculer sur un axe commun (90), qui est fixé à l'anneau (20) de valve, les deux ailes (50,70) de valve pouvant prendre, en fonction du sens de l'écoulement du sang, une position ouverte sensiblement verticale par rapport au plan (44) de l'anneau de valve ou une position fermée sensiblement en direction du plan (44) de l'anneau de valve, dans laquelle les deux ailes (50,70) de valve ferment pratiquement complètement, par leurs surfaces (55, 75) prises ensemble le canal (24) d'écoulement,
caractérisée en ce que
l'axe (90) est disposé de manière excentrée c'est-à-dire parallélement et à distance d'un diamètre (43) de cercle sur l'anneau (20) de valve et l'une (70) des ailes de valve a une plus grande surface (75) que l'autre aile (50) de valve.

2. Prothèse de valve du coeur à aile double suivant la revendication 1,
caractérisé en ce que
l'excentricité de l'axe (90), c'est-à-dire la distance d'un axe (91), au milieu dans le sens de la longueur, de l'axe (90) à un diamètre (43) de l'anneau (20) de valve représente environ de 4 à 22% de la longueur d'un diamètre intérieur de l'anneau (20) de valve.

3. Prothèse de valve du coeur à aile double suivant la revendication 1 ou 2,
caractérisée en ce que
le canal (24) d'écoulement a une section transversale (25);
l'aile (70) de valve de plus grande surface ferme environ 55 à 80% de la section transversale (25) du canal d'écoulement et
l'aile (50) de valve de plus petite surface ferme environ 45 à 20% de la section transversale du canal d'écoulement.

4. Prothèse de valve du coeur à aile double suivant la revendication 3,
caractérisée en ce que
l'aile (70) de valve de plus grande surface ferme environ de 60 à 68% de la section transversale (25) du canal d'écoulement et
l'aile (50) de valve de plus petite surface ferme environ 40 à 32% de la section transversale (25) du canal d'écoulement.

5. Prothèse de valve du coeur à aile double suivant l'une des revendications 1 à 4,
caractérisée en ce que
l'aile (70) de valve de plus grande surface est disposée de manière dorsale pour l'utilisation en tant que prothèse aortique.

6. Prothèse de valve du coeur à aile double suivant l'une des revendications 1 à 4,
caractérisée en ce que
l'aile (70) de valve de plus grande surface est disposée de manière dorsale pour l'utilisation en tant que prothèse mitrale.

7. Prothèse de valve du coeur à aile double suivant l'une des revendications 1 à 6,
caractérisée en ce que
l'axe (90) comporte deux tronçons (93,97) d'extrémité; chaque tronçon (93,97) d'extrémité de l'axe est maintenu dans un palier d'axe, qui est sous la forme d'un évidement dans l'anneau (20) de valve; chaque palier d'axe est sous la forme d'un évidement (36) en longueur, qui s'étend dans une direction allant de la gauche en bas, vers la droite en haut par rapport au plan (44) de l'anneau de valve.

8. Prothèse de valve du coeur à aile double suivant la revendication 7,
caractérisée en ce que
la circonférence intérieure (21) de l'anneau (20) de valve augmente au moins dans la région des deux paliers (36) d'axe dans le sens d'écoulement du sang.

9. Prothèse de valve du coeur à aile double suivant la revendication 7 ou 8
caractérisée en ce que
l'anneau (20) de valve a une plus grande hauteur et/ou un évasement dans la région des paliers (36) d'axe.

10. Prothèse de valve du coeur à aile double suivant l'une des revendications 1 à 9,
caractérisée en ce que,
l'axe (90) est en deux parties et est constitué de deux tronçons (92,96) d'axe;
chaque tronçon (92,96) d'axe comporte à une extrémité une tête (93,96) épaissie, qui est maintenue dans le palier (35,36) d'axe; les deux autres tronçons d'extrémité (92,96) d'axe sont sous la forme d'une broche (98) et d'une douille (99), qui peuvent être enfichées l'une dans l'autre.

11. Prothèse de valve du coeur à aile double suivant l'une des revendications 1 à 10,
caractérisée en ce que
chaque aile (50,70) de valve est semi-lunaire et comporte une partie de bord (52,72) courbe et une partie de bord (54,74) droite;
au moins un tronçon (64, 84) de douille, dans le perçage (65,85) duquel l'axe (90) passe, est issu de la partie de bord droite (54,74) en étant d'une seule pièce avec elle;
le diamètre extérieur de l'axe (90) et le diamètre intérieur du perçage (65,85) des tronçons (64,84) de douille sont tels que d'une part une possibilité de libre basculement des ailes (50,70) de valve autour de l'axe (90) soit garantie et que d'autre part du sang ne puisse normalement pas pénétrer dans le perçage (65,85) des tronçons (64,84) de douilles.

12. Prothèse de valve du coeur à aile double suivant l'une des revendications 1 à 11,
caractérisée en ce que
chaque aile (50,70) de valve est semi-lunaire et comporte une partie de bord (52,72) courbe et une partie de bord (54,74) droite
au moins un tronçon (64,84) de douille, dans le perçage (65,85) duquel l'axe (90) passe, est issu de la partie de bord droite (54,74) en étant d'une seule pièce avec elle;
chaque aile (50,70) de valve a, du côté (57,77) d'admission de l'écoulement, une configuration convexe du sommet (53,73) de la partie de bord (52,72) courbe jusqu'à la partie de bord droite (54,74), cette configuration convexe continuant dans la courbure du tronçon (64,84) de douille.

13. Prothèse de valve du coeur à aile double suivant l'une des revendications 1 à 12,
caractérisée en ce que
les deux ailes (50,70) de valve délimitent, dans la position ouverte, un angle α d'ouverture d'ailes d'environ 10 à 40°, qui est délimité pour chacune des ailes par une droite (47,48), laquelle coupe l'axe (91), au milieu dans le sens de la longueur, de l'axe (90) et passe par le sommet (53,73) sur la partie de bord (52,72) courbe de chaque aile (50,70) de valve.

14. Prothèse de valve du coeur à aile double suivant la revendication 13,
caractérisée en ce que
les deux ailes (50,70) de valve délimitent, dans la position ouverte, un angle α d'ouverture d'ailes d'environ 12 à 18°.

15. Prothèse de valve du coeur à aile double suivant l'une des revendications 1 à 14,
caractérisée en ce que
les ailes (50,70) de valve prennent, dans la position ouverte, une position qui correspond sensiblement à la direction des lignes de l'écoulement du sang.

16. Prothèse de valve du coeur à aile double suivant l'une des revendications 1 à 15,
caractérisée en ce que
des butées, qui empêchent les ailes (50,70) de valve de sortir, dans la position fermée, de leur position prévue sont formées sur la circonférence intérieure (21) de l'anneau (20) de valve;
ces butées sont chacune sous la forme de deux parties (22,22') de gradin, qui s'étendent des deux côtés d'un palier (35,36) d'axe sur une distance relativement courte de la circonférence intérieure (21) sensiblement en direction du plan (44) de l'anneau de la valve.

17. Prothèse de valve du coeur à aile double suivant la revendication 16,
caractérisée en ce que
chaque partie (22,22') de gradin a une longueur d'environ 3 à 6mm.

18. Prothèse de valve du coeur à aile double suivant la revendication 16 ou 17,
caractérisée en ce que
chaque partie (22,22') du gradin s'étend dans une direction, qui va du palier (35) d'axe au bord supérieur (distal) de l'anneau (20) de valve dans le sens de l'écoulement.

19. Prothèse de valve du coeur à aile double suivant l'une des revendications 1 à 18,
caractérisée en ce que
des butées qui empêchent les ailes (50,70) de valve de sortir, en position ouverte, de leur position prévue, sont formées sur la périphérie intérieure (21) de l'anneau (20) de valve;
ces butées sont sous la forme de deux broches (23) qui sont en saillie dans la région supérieure (distale), dans le sens de l'écoulement, de l'anneau (20) de valve de la circonférence (21) vers l'intérieur.

20. Prothèse de valve du coeur à aile double suivant l'une des revendications 1 à 19,
caractérisée en ce que
l'anneau (20) de valve comporte au moins une partie (34') d'anneau de valve ayant une hauteur plus grande;
cette partie (34') d'anneau de valve est voisine de l'aile (50) de valve de plus petite surface.

21. Prothèse de valve du coeur à aile double suivant l'une des revendications 1 à 20,
caractérisée en ce que
l'anneau (20) et /ou les ailes (50,70) de valve ont une structure à plusieurs couches, comportant une couche de couverture en carbone pyrolytique, une ou plusieurs couches intermédiaires en carbure (s) de titane et un noyau en titane métallique.
